# EUROPÄISCHE PATENTANMELDUNG

(11) **EP 3 154 278 A1**
(43) Veröffentlichungstag der Anmeldung: **12.04.2017**
(21) Anmeldenummer: 16189577.6
(22) Anmeldetag: 20.09.2016
(51) Int. Cl.: H04R 25/00, A61B 5/024, A61B 5/11

(54) **HÖRSYSTEM UND VERFAHREN ZUM BETRIEB EINES HÖRSYSTEMS**

(30) Priorität: 09.10.2015 DE 102015219573
(71) Anmelder: Sivantos Pte. Ltd., Singapore 139959 (SG)
(72) Erfinder: ASCHOFF, Stefan, 90542 Eckental (DE); CLAD, Lars, 90433 Nürnberg (DE)
(74) Vertreter: FDST Patentanwälte

(57) **Zusammenfassung**

Es wird ein Hörsystem (2) für einen Anwender angegeben, mit einem Hörgerät (4), insbesondere einem BTE-Gerät, welches ein Mikrofon (6) aufweist, zur Erfassung eines Audiosignals, und mit einer Steuereinheit (12), die ausgebildet ist, auf Grundlage des Audiosignals einen physiologischen Zustand (Z) des Anwenders zu klassifizieren. Das Audiosignal wird insbesondere hinsichtlich charakteristischer Merkmale untersucht, die eine Erkennung bestimmter physiologisch relevanter Geräusche des Anwenders ermöglichen. Hieraus wird dann das Vorliegen von Symptomen (S1, S2, S3) abgeleitet, welche einen bestimmten Zustand (Z) charakterisieren.

## Beschreibung

Die Erfindung betrifft ein Hörsystem sowie ein Verfahren zum Betrieb eines Hörsystems.

Hörsysteme weisen regelmäßig ein oder zwei Hörgeräte auf, die häufig von einem Anwender mit einer Hörschädigung zur Verstärkung von Umgebungsgeräuschen getragen werden. Dabei wird ein jeweiliges Hörgerät üblicherweise in oder am Ohr getragen und weist zumindest ein Mikrofon auf, zur Aufnahme von Umgebungsgeräuschen, welche dann in geeigneter Weise verstärkt werden und an einen Lautsprecher oder Hörer des Hörgeräts zur Ausgabe weitergeleitet werden. Die Ausgabe erfolgt typischerweise in Richtung oder innerhalb des Gehörgangs. Die Verstärkung und die Ausgabe stellen dabei eine Grundfunktionalität des Hörsystems dar.

Zur Erweiterung des Funktionsumfangs eines Hörsystems über die Grundfunktionalität hinaus ist es grundsätzlich bekannt, zusätzliche Sensoren, d.h. Zusatzsensoren zu integrieren, mittels derer physiologische Parameter, wie z.B. Körpertemperatur oder Blutsauerstoffgehalt, des Anwenders gemessen werden, um bei Überschreitung bestimmter Grenzwerte einen Alarm auszulösen und auf diese Weise eine Gesundheitsüberwachung zu realisieren.

Vor diesem Hintergrund ist es eine Aufgabe der Erfindung, ein verbessertes Hörsystem anzugeben.

Die Aufgabe wird erfindungsgemäß gelöst durch ein Hörsystem mit den Merkmalen gemäß Anspruch 1 sowie durch ein Verfahren mit den Merkmalen gemäß Anspruch 15. Vorteilhafte Ausgestaltungen, Weiterbildungen und Varianten sind Gegenstand der Unteransprüche. Dabei gelten die Ausführungen im Zusammenhang mit dem Hörsystem sinngemäß auch für das Verfahren und umgekehrt.

Das Hörsystem ist zum Tragen durch einen Anwender ausgebildet. Zudem ist das Hörsystem binaural ausgebildet, mit zwei Hörgeräten, welche jeweils ein Mikrofon aufweisen zur Erfassung eines Audiosignals. Genauer wird darunter insbesondere verstanden, dass beide Mikrofone ein Geräusch, d.h. insbesondere ein Audiosignal aus der Umgebung, aufnehmen und daraus jeweils eine elektrische Version ebenjenes Geräusches, d.h. ein Audiosignal erzeugen. Die beiden Mikrofone erzeugen jeweils ein Audiosignal, d.h. es werden zwei Audiosignale erzeugt, welchen insbesondere dasselbe Geräusch zugrunde liegt.

Das Hörsystem weist weiterhin eine Steuereinheit auf, die ausgebildet ist, auf Grundlage eines Audiosignals einen physiologischen Zustand des Anwenders zu klassifizieren. Vorliegend ist die Steuereinheit zudem ausgebildet, auf Grundlage der beiden Audiosignale, genauer gesagt der beiden von den Mikrofonen erfassten Audiosignale, einen Eigenanteil, der vom Anwender stammt, hervorzuheben, d.h. insbesondere zu ermitteln und gegenüber anderen Anteilen, d.h. NichtEigenanteilen, zu verstärken, und zur Klassifizierung des physiologischen Zustands zu verwenden. Dieser Ausgestaltung liegt insbesondere der Gedanke zugrunde, dass diese sogenannte binaurale Verrechnung eine verbesserte Unterscheidung zwischen Umgebungsgeräuschen und vom Anwender erzeugten Geräuschen ermöglicht. Zur Durchführung der binauralen Verrechnung umfasst die Steuereinheit insbesondere eine binaurale Verrechnungseinheit.

Der Erfindung liegt insbesondere der Gedanke zugrunde, dass zur Klassifizierung eines physiologischen Zustands des Anwenders nicht notwendigerweise Zusatzsensoren verwendet werden müssen, sondern die Messung und Bestimmung relevanter physiologischer Parameter als Grundlage zur Erkennung des Zustands auch mittels des Mikrofons möglich ist, welches ohnehin zur Realisierung der Grundfunktionalität des Hörgeräts vorhanden ist. Zusätzlich zur Verarbeitung der aufgenommenen Audiosignale zwecks Verstärkung und Ausgabe an den Anwender, erfolgt erfindungsgemäß somit zusätzlich eine insbesondere separate Verarbeitung der erfassten Audiosignale zur Klassifizierung des physiologischen Zustands des Anwenders. Das Hörsystem ist dann vorteilhaft als insbesondere automatisiertes Diagnosesystem ausgebildet, wobei unter Diagnose zunächst allgemein die Identifizierung eines bestimmten Zustands aufgrund von ausgewerteten Signalen verstanden wird, unabhängig davon, ob dieser Zustand mit einer Erkrankung korrespondiert. Zur Klassifizierung werden dann insbesondere die vom Mikrofon erfassten Audiosignale hinsichtlich physiologisch relevanter Geräusche untersucht, d.h. von der Steuereinheit, die hier insbesondere als Klassifikator wirkt, analysiert, und auf Grundlage dessen dann ein physiologischer Zustand klassifiziert.

Ein besonderer Vorteil der Erfindung besteht somit insbesondere darin, dass das Hörsystem zusätzlich zur Grundfunktionalität der Verstärkung von Umgebungsgeräuschen auch eine Überwachung und Bewertung des allgemeinen Befindens des Anwenders durchführt, indem die erfassten Audiosignale zusätzlich zur Erkennung eines physiologischen Zustand des Anwenders herangezogen werden. Das Mikrofon des Hörgeräts erfüllt hierbei insbesondere eine vorteilhafte Doppelfunktion, sodass auf die Verwendung von speziellen Sensoren zur Messung der physiologischen Parameter zwecks Klassifizierung eines physiologischen Zustands des Anwenders zunächst verzichtet werden kann.

Ein physiologischer Zustand ist allgemein ein körperlicher Zustand des Anwenders, welcher sich insbesondere in einer Anzahl von Symptomen äußert. Mit anderen Worten: Der physiologische Zustand des Anwenders ist Ursache für eine Anzahl von Symptomen, deren Vorliegen dann vom Hörsystem durch Analyse der vom Mikrofon erfassten Audiosignale erkannt wird. Mittels der erkannten Symptome erfolgt dann die Klassifizierung derart, dass das Ergebnis der Analyse einen Schluss auf den Zustand zulässt.

Die Symptome selbst werden aus dem Audiosignal oder mehreren Audiosignalen bestimmt. In dieser Hinsicht erfolgt dann insbesondere eine Art zweistufige Klassifizierung, bei der in einem ersten Schritt die Symptome auf Basis der Audiosignale klassifiziert werden und in einem zweiten Schritt dann der Zustand ausgehend von den erkannten Symptomen klassifiziert wird.

Unter Klassifizierung wird hier allgemein das Einteilen einer gegebenen Menge nach aufgestellten Klassen verstanden und im Besonderen eine Untersuchung eines Signals auf bekannte, hinterlegte Muster, mit einer anschließenden Zuordnung des Signals zu einer bestimmten Klasse, welche durch diese Muster charakterisiert ist. Üblicherweise wird dabei das Signal auf eine Anzahl von Merkmalen, d.h. Muster, untersucht, welche möglichst einzigartig für eine bestimmte Klasse sind und dann eine entsprechende Zuordnung ermöglichen. Alternativ oder zusätzlich ist das Signal durch ein bestimmtes Ereignis verursacht, wobei dann das Ereignis ein Signal mit klassenspezifischen Merkmalen verursacht. Das Ereignis wird dann klassifiziert, indem dieses als Ereignis einer bestimmten, vorbekannten Klasse identifiziert wird. Im Hörgerätebereich erfolgt beispielsweise häufig eine Klassifizierung von Audiosignalen, insbesondere Umgebungsgeräuschen zwecks Zuordnung des Umfelds des Anwenders zu einer bestimmten Geräuschsituation, auch als Szene bezeichnet. Solche Szenen als Klassen sind beispielsweise Sprache in ruhiger Umgebung oder Sprache in geräuschreicher Umgebung. Diese Art der Klassifizierung ist beispielsweise beschrieben in Hamacher et al. "Signal Processing in High-End Hearing Aids: State of the Art, Challenges, and Future Trends" EURASIP Journal on Applied Signal Processing 2005.

Vorliegend erfolgt eine Klassifizierung zur Erkennung eines physiologischen Zustands des Anwenders. Entsprechend existiert eine Anzahl solcher Zustände, wobei jeder Zustand durch vorbestimmte Merkmale gekennzeichnet ist, insbesondere durch eine Anzahl an Symptomen, welche eine Folge des Zustands sind. Diese Symptome stellen wiederum selbst jeweils ein Klasse dar und lassen sich durch entsprechende Merkmale voneinander unterscheiden. Im Falle einer Analyse von Audiosignalen sind die Symptome entsprechende Geräusche, mit beispielsweise charakteristischem Frequenzspektrum oder Amplitudenverlauf. So sind beispielsweise Niesen, Husten oder Stimmverzerrungen, d.h. insbesondere eine Verschiebung der Stimme hin zu tieferen Frequenzen, ausgehend von einem Audiosignal erkennbar und bilden jeweils ein Symptom, d.h. eine Symptom-Klasse, welcher das Audiosignal zugeordnet wird. Das zugehörige Geräusch wird also als ein Symptom klassifiziert. Diese Klassifizierung erfolgt insbesondere anhand im Hörgerätebereich an sich bekannter Verfahren und Algorithmen, insbesondere unter Verwendung sogenannter Bayes'scher Netze. Solche Verfahren sind beispielsweise in Taras Kowaliw, Nicolas Bredeche, Rene Doursat: "Growing Adaptive Machines: Combining Development and Learning in Artificial Neural Networks" (Studies in Computational Intelligence) Springer-Verlag Berlin Heidelberg 2014 beschrieben.

Ein bestimmter Zustand ist nun durch eine Anzahl charakteristischer Symptome definiert und wird entsprechend klassifiziert, indem die auf Grundlage des Audio-signals ermittelten Symptome zur Zuordnung zu einem bestimmten, vordefinierten Zustand, d.h. einer Zustands-Klasse verwendet werden. So ist beispielsweise eine Erkältung als Zustand durch ein Niesen als Symptom charakterisiert. Wird also durch Analyse des Audiosignals ein Niesen erkannt, wird entsprechend der Zustand als Erkältung klassifiziert. Grundsätzlich ist auch das vorliegen mehrerer Zustände gleichzeitig möglich. Daher ist die Steuereinheit zweckmäßigerweise zur Klassifizierung mehrerer gleichzeitig vorliegender Zustände ausgebildet.

Die Steuereinheit ist entweder im Hörgerät, insbesondere in dessen Gehäuse, untergebracht oder außerhalb des Hörgeräts in einem externen Gerät, d.h. intern bzw. extern angeordnet. Eine interne Steuereinheit führt insbesondere zu einer besonders kompakten Ausführung des Hörsystems, wohingegen eine externe Steuereinheit den Vorteil hat, besonders leistungsfähig zu sein, da hierbei insbesondere eine separate Stromversorgung ausgebildet ist. In jedem Fall ist die Steuereinheit mit dem Mikrofon derart verbunden, dass die erfassten Audiosignale an die Steuereinheit weitergegeben werden, um anschließend von dieser analysiert zu werden.

Das Hörgerät ist insbesondere ein BTE-Gerät, d.h. ein Hörgerät mit einem Gehäuse, welches hinter dem Ohr getragen wird. In dem Gehäuse sind dann die meisten wesentlichen Komponenten des Hörgeräts untergebracht, insbesondere eine oder eine Kombination folgender Komponenten: Batterie, Mikrofon, Elektronik, Bedienelemente und Programmierschnittstelle. Die Schallausgabe erfolgt über einen Hörer, welcher je nach Ausgestaltung des BTE-Geräts entweder ebenfalls im Gehäuse untergebracht ist oder als Teil eines Ohrstücks über ein Kabel mit dem Gehäuse verbunden ist und zum Tragen in den Gehörgang eingesetzt wird. Grundsätzlich ist jedoch auch eine Ausbildung des Hörgeräts als sogenanntes ITE-Gerät, d.h. vollständig oder überwiegend im Ohr getragenes Hörgerät denkbar.

Grundsätzlich sind zur Bestimmung eines physiologischen Zustands vorrangig die vom Anwender generierten Geräusche relevant, d.h. die Anwendergeräusche, während die Umgebungsgeräusche üblicherweise keinen Schluss auf den Zustand des Anwenders zulassen. So ist es beispielsweise für den Zustand des Anwenders nicht unmittelbar relevant, ob jemand anderes niest. Die binaurale Verrechnung ermöglicht jedoch vorteilhaft eine entsprechende Unterscheidung und Zuordnung eines Geräuschs zum Anwender. Durch die Ermittlung des Eigenanteils mittels binauraler Verrechnung werden somit auf vorteilhafte Weise genau die zur Klassifizierung relevanten Geräusche hervorgehoben. Dieses Ermitteln des Eigenanteils erfolgt insbesondere entgegen der herkömmlichen Verwendung einer binauralen Verrechnung, da bei dieser herkömmlichen Verwendung gezielt Geräusche aus der Umgebung hervorgehoben werden. Besonders in schwierigen Hörsituationen, beispielsweise den sogenannten "Cocktail Party Situationen" mit vielen Sprechern in der nahen Umgebung, wird die herkömmliche binaurale Verrechnung genutzt, um nach Richtung und Frequenzanteilen auf einzelne Sprecher zu fokussieren, d.h. eine räumliche Fokussierung auf bestimmte Geräuschquellen vorzunehmen. Mittlerweile werden hierfür auch Apps angeboten, die dem Anwender die Ausrichtung binaural arbeitenden Richtmikrofone seines Hörsystems ermöglichen. Während die herkömmliche binaurale Verrechnung nun je nach Situation Geräusche aus einer beliebigen Richtung hervorhebt, hebt die hier eingesetzte binaurale Verrechnung vorzugsweise konstant die Geräusche aus der vorderen Richtung und damit die Anwendergeräusche hervor, um diese anschließend zwecks Klassifizierung zu verwerten.

Insbesondere vor dem Hintergrund, dass die vom Anwender selbst erzeugten Geräusche besonders zur Klassifizierung eines physiologischen Zustands geeignet sind, wird zur Erfassung des Audiosignals zweckmäßigerweise ein Körperschallmikrofon verwendet. Daher weist in einer geeigneten Ausgestaltung das Hörsystem ein Körperschallmikrofon auf zur Erfassung eines Anwendergeräusches als Audiosignal, das vom Anwender verursacht ist und insbesondere einer Gruppe von Geräuschen angehört, umfassend: Atemgeräusch, Atemfrequenz, Trittschall, Körperschall, Puls, d.h. insbesondere Pulsfrequenz, Stimme und Zähneknirschen. Im Gegensatz zur oben erwähnten Bestimmung des Eigenanteils aus den Audiosignalen erfolgt die Erfassung von Anwendergeräuschen mittels des Körperschallmikrofons dann direkt, d.h. das Körperschallmikrofon ist besonders zur Aufnahme und Erfassung von Anwendergeräuschen ausgebildet, und liegt dazu beispielsweise direkt an der Haut des Anwenders an, wenn das Hörgerät getragen wird. Insbesondere weist das Körperschallmikrofon seitlich vom Ohrstück weg und auf die Gehörgangwand zu, im Gegensatz zu einem regulären Mikrofon eines ITE-Geräts, bei dem das Mikrofon zur Erfassung von Umgebungsgeräuschen üblicherweise aus dem Gehörgang hinaus weist. Indem das Körperschallmikrofon direkt am Körper des Anwenders anliegt oder z.B. über Gehäuseteile eines Ohrstückes am Körper anliegt, werden Umgebungsgeräusche in entsprechend sehr geringem Maße erfasst.

Die Erfassung der genannten Anwendergeräusche ermöglicht insbesondere eine Bestimmung physiologischer Parameter des Anwenders. So ermöglicht die Analyse des Atemgeräuschs die Bestimmung einer Atemfrequenz des Anwenders als Parameter. Die Messung von Trittschall ermöglicht eine Messung der Schrittzahl des Anwenders als Parameter und damit Rückschlüsse auf die Bewegungsaktivität des Anwenders. Unter Körperschall werden dann insbesondere solche Geräusche verstanden, die gewollt oder ungewollt vom Körper des Anwenders erzeugt werden, beispielsweise Niesen, Husten, Räuspern oder Verdauungsgeräusche. Durch eine Pulsmessung ist es dann möglich, die Pulsfrequenz als Parameter des Anwenders zu ermitteln. Unter Stimme wird in diesem Zusammenhang insbesondere verstanden, dass erfasst wird, wie viel der Anwender selbst spricht und insbesondere in Kommunikation mit anderen Menschen steht. Insbesondere wird dazu als Parameter die Redezeit des Anwenders erfasst. Mittels dieser Anwendergeräusche erfolgt dann durch die Steuereinheit die Bestimmung und insbesondere auch Quantifizierung physiologisch relevanter Parameter des Anwenders. Insgesamt weist jedes Anwendergeräusch insbesondere ein charakteristisches Frequenzspektrum auf, aufgrund dessen ein jeweiliges Anwendergeräusch mittels der Steuereinheit aus dem Audiosignal erkannt wird. Das entsprechende Frequenzspektrum bildet dabei sozusagen einen Fingerabdruck eines jeweiligen Geräuschs. Die Erkennung solcher Fingerabdrücke und Zuordnung zu einem bestimmten Typ von Anwendergeräusch erfolgt insbesondere aufgrund herkömmlicher Algorithmen, wie beispielsweise beschrieben in: Richard O. Duda, Peter E. Hart, David G. Stork, "Pattern Classification", John Wiley & Sons, Inc., 2001, beschrieben. Die charakteristischen Anwendergeräusche stellen somit insbesondere Symptome dar, welche entsprechend klassifiziert werden.

In einer besonders geeigneten Variante sind das Mikrofon und das Körperschallmikrofon als separate Komponenten des Hörgeräts ausgebildet. Die Grundfunktion und die Zusatzfunktion des Hörsystems sind auf diese Weise durch entsprechend spezialisierte Mikrofone realisiert. So dient das Mikrofon vorrangig zur Aufnahme von Umgebungsgeräuschen zwecks Verstärkung und Ausgabe über den Hörer und das Körperschallmikrofon dient vorrangig zur Erfassung und Registrierung von Anwendergeräuschen, welche insbesondere nicht an den Anwender weitergegeben werden, sondern von der Steuereinheit zur Klassifizierung eines physiologischen Zustands verwendet werden. Dazu ist das Mikrofon insbesondere nach außen gerichtet und sozusagen als freies Mikrofon am Hörgerät angeordnet, um auf optimale Weise Umgebungsgeräusche aufzunehmen und eine Erfassung von Anwendergeräuschen zu vermeiden. Bei einem BTE-Gerät ist dann das Mikrofon insbesondere im Gehäuse angeordnet und weist in die Umgebung des Anwenders, beispielsweise in Blickrichtung. Das Körperschallmikrofon ist dagegen zum Anwender hin gerichtet und steht insbesondere in Kontakt mit dessen Körperoberfläche, insbesondere dessen Haut. Dadurch liegt das Körperschallmikrofon dann am Anwender an und nimmt vorrangig Anwendergeräusche auf, wohingegen Umgebungsgeräusche weitestgehend nicht erfasst werden. Durch diese spezialisierte Ausgestaltung mit unterschiedlichen Mikrofonen ist dann insbesondere eine verbesserte Trennung zwischen internen und externen Geräuschen realisiert. Dies ist insbesondere bei einem nicht-binauralen Hörsystem von Bedeutung, aber auch bei einem binauralen System vorteilhaft.

In einer vorteilhaften Weiterbildung weist das Hörgerät ein Ohrstück auf zum Einsetzen in einen Gehörgang des Anwenders, und das Körperschallmikrofon ist ein Teil des Ohrstücks. Mit anderen Worten: Beim Tragen des Hörsystems ist das Körperschallmikrofon in den Gehörgang des Anwenders eingesetzt, wodurch auf besonders optimale Weise Anwendergeräusche erfasst werden, ohne dass zusätzlich möglicherweise störende Umgebungsgeräusche erfasst werden.

Die Steuereinheit ist zur Klassifizierung eines physiologischen Zustands ausgebildet, der vorzugsweise einer Gruppe von Zuständen angehört, umfassend: Erkältung, Sturz, Schlafverhalten, soziale Aktivität, Bewegungsaktivität, Stresslevel, Atemprobleme. Der physiologische Zustand ist somit insbesondere ein Gesundheitszustand des Anwenders, dabei jedoch nicht notwendigerweise ein pathologischer Zustand. Vielmehr ist der physiologische Zustand ein Ausdruck des Allgemeinbefindens des Anwenders, lässt dabei aber möglicherweise Rückschlüsse auf eventuelle Krankheiten oder Beschwerden des Anwenders zu. Ein jeweiliger physiologischer Zustand ist insbesondere Ursache für ein entsprechendes Verhalten des Anwenders, welches sich insbesondere in bestimmten, charakteristischen Anwendergeräuschen äußert.

Ein physiologischer Zustand wird insbesondere durch eine Auswertung von Eigenschaften und/oder Werten eines oder mehrerer physiologischer Parameter bestimmt. So ist beispielsweise eine Erkältung eine Ursache für häufiges Niesen, welches sich in entsprechenden Anwendergeräuschen äußert und dann mittels des Mikrofons erfasst wird. Aufgrund der erkannten Häufigkeit der Niesgeräusche kann dann auf eine Erkältung geschlossen werden, d.h. der physiologische Zustand des Anwenders wird als Erkältung klassifiziert. Dabei ist die Steuereinheit vorzugsweise derart ausgebildet, dass eine entsprechende Differenzierung zwischen vergleichsweise häufigem, d.h. insbesondere in Zeitabständen geringer als ein Tag, und lediglich gelegentlichem Niesen, d.h. insbesondere in Zeitabständen größer als einem Tag, erfolgt, wobei dann eine Unterscheidung zwischen einer Erkältung und anderen Niesreizen möglich ist.

Ein Sturz wird dann beispielsweise durch entsprechende Aufschlaggeräusche erkannt. Ein bestimmtes Schlafverhalten, beispielsweise unruhiger Schlaf, ist durch Überprüfung von Atemgeräuschen und Puls klassifizierbar, vorzugsweise in Kombination mit zusätzlich erfassten Umgebungsgeräuschen. Die soziale Aktivität des Anwenders ist durch Messung der Redezeit des Anwenders, insbesondere in Kombination mit von anderen Menschen aufgenommenen Stimmen klassifizierbar. Die Bewegungsaktivität des Anwenders wird insbesondere durch Erfassung und Analyse des Trittschalls und allgemein der Schrittzahl des Anwenders sowie dessen Puls bewertet. Stresslevel und Atemprobleme sind insbesondere Ursache für charakteristisch veränderten Puls und Atmung, welche entsprechend mittels des Mikrofons messbar sind. Insgesamt erfolgt also eine Quantifizierung physiologischer Parameter des Anwenders zwecks Erkennung und Bewertung diverser Symptome, welche sich wiederum als Folgen bestimmter physiologischer Zustände ergeben.

Die Zuverlässigkeit, mit der physiologische Parameter der Atemwegsorgane des Anwenders erfasst und ausgewertet werden, ist in einer vorteilhaften Ausgestaltung durch den Einsatz von Körperschallmikrofonen weiter verbessert. Durch eine geeignete gemeinsame Auswertung der Audiosignale eines Körperschallmikrofons mit denjenigen Audiosignalen, die aus der binauralen Verrechnung gewonnen werden, hebt den Eigenanteil in vorteilhafter Weise noch einmal hervor. Beispielsweise nehmen die Körperschallmikrofone nicht nur Geräusche der Atemwegsorgane auf, sondern auch andere im oder am Körper verursachte Geräusche wie etwa Trittschall, Puls, Zähneknirschen oder Verdauungsgeräusche. Die aus der binauralen Verrechnung gewonnenen Audiosignale heben zwar die Geräusche aus der vorderen Richtung, aus welcher die Anwendergeräusche kommen, hervor, diese können aber unter Umständen auch von Umgebungsgeräuschen überlagert sein, welche von einem Körperschallmikrofon jedoch nicht erfasst werden. Eine geeignete Kombination beider Audiosignale, d.h. des Audiosignals von einem Mikrofon und dem Audiosignal von einem als Körperschallmikrofon ausgebildeten Zusatzsensor, hebt die gleichen Anteile hervor, nämlich insbesondere die Anwendergeräusche, und führt daher zu einer Hervorhebung der von den Atemwegsorganen verursachten Geräusche, während die übrigen Anteile abgeschwächt werden. Die auf diese Weise zweifach ermittelten physiologischen Parameter werden daher vorteilhaft mit höherer Genauigkeit ermittelt und eine anschließende Klassifikation eines Symptoms auf Grundlage dieses Parameters erfolgt umso zuverlässiger.

Zur Hervorhebung von Anwendergeräuschen, d.h. des Eigenanteils der Audiosignale, werden in einer vorteilhaften Weiterbildung eines Hörsystems mit zusätzlichem Körperschallmikrofon das Audiosignal des regulären Mikrofons und das Audiosignal des Körperschallmikrofons miteinander kombiniert. Hierzu weist die Steuereinheit eine Audiosignalkombinationseinheit auf.

Bei einem binauralen Hörsystem werden die Audiosignale der beiden Mikrofone der zwei Hörgeräte insbesondere zu einem Kombinationssignal zusammengefasst, insbesondere mittels der binauralen Verrechnungseinheit. Das Kombinationssignal weist dann den hervorgehobenen Eigenanteil auf, ist also ein Audiosignal mit hervorgehobenem Eigenanteil. In einer vorteilhaften Ausgestaltung des Hörsystems weist die Steuereinheit eine Audiosignalkombinationseinheit auf, welche das Kombinationssignal mit dem Audiosignal eines Körperschallmikrofons des Hörsystems kombiniert, zur weiteren Hervorhebung des Eigenanteils. Allgemein erfolgt mittels der Audiosignalkombinationseinheit demnach eine Kombination zweier Audiosignale unterschiedlicher Mikrofone, nämlich eines regulären, nach außen gerichteten Mikrofons und eines Körperschallmikrofons. Mit anderen Worten: die Audiokombinationseinheit dient der Kombination und insbesondere dem Vergleichen zweier Audiosignale, die von Mikrofonen unterschiedlicher Art stammen und dabei Geräusche aus unterschiedlichen Geräuschquellen unterschiedlich stark erfassen. Insbesondere wird hierbei das Audiosignal der binauralen Verrechnungseinheit, welches ein Kombinationssignal der Audiosignale der beiden regulären Mikrofone ist und einen bereits hervorgehobenen Eigenanteil aufweist, mit dem Audiosignal des Körperschallmikrofons, welches bereits aufgrund der Positionierung hauptsächlich Anwendergeräusche erfasst, kombiniert. Aufgrund der Kombination ist es dann möglich, in oben beschriebener Weise die Anwendergeräusche gegenüber anderen Geräuschen weiter hervorzuheben und eine zuverlässigere Klassifizierung des physiologischen Zustands zu gewährleisten.

In einer geeigneten Ausführungsform nimmt die Audiosignalkombinationseinheit eine Kombination vor, indem ebenjene die beiden Audiosignale der unterschiedlichen Mikrofone addiert. Im oben genannten Beispiel der Geräusche der Atemwegsorgane werden ebendiese Geräusche dann um 3dB stärker hervorgehoben als die Signale anderer, insbesondere unkorrelierter Geräusche.

Eine weitere geeignete Möglichkeit ist die Durchführung einer Kreuzkorrelation der beiden Audiosignale, wodurch ein Korrelationsergebnis gewonnen wird, welches anschließend genutzt wird, um ein zusätzliches adaptives Filter einzustellen. Die Einstellung erfolgt beispielsweise derart, dass das Filter dann gerade diejenigen Frequenzen hervorhebt, die Bestandteil beider Audiosignale sind, und andere Frequenzen dagegen abschwächt. Alternativ werden aus den beiden Audiosignalen ein Summensignal und ein Differenzsignal berechnet, wobei das Differenzsignal dann entsprechend die nicht-gleichartigen Signalanteile enthält. Daher lässt sich das Differenzsignal besonders geeignet zur Einstellung eines adaptiven Filters nutzen, welches die entsprechenden Anteile des Summensignales noch einmal abschwächt.

Auf Basis der erfolgten Klassifizierung eines entsprechenden physiologischen Zustands des Anwenders sind generell verschiedene Reaktionen denkbar. In einer ersten Variante erfolgt lediglich eine Klassifizierung des Zustands und Weitergabe dieser Klassifizierung an den Anwender zu Informationszwecken. In einer zweiten Variante werden die entsprechenden Erkenntnisse gespeichert und stehen beispielsweise bei einem Arztbesuch als Grundlage für eine Diagnose zur Verfügung. In einer dritten Variante umfasst das Hörsystem insbesondere zusätzlich ein Notfallsystem, bei dem bei einer Erkennung bestimmter physiologischer Zustände ein Notruf abgesetzt wird. Beispielsweise wird aufgrund der Erfassung und Analyse des Pulses und der Atemfrequenz des Anwenders ein physiologischer Zustand als Schlaganfall klassifiziert und als Reaktion hierauf mittels der Steuereinheit ein Notruf abgesetzt, um dem Anwender möglichst schnell geeignete Hilfe zukommen zu lassen. In einer weiteren Variante wird die Klassifizierung im Rahmen eines Trainingsprogramms, beispielsweise eines Ausdauertrainings verwendet, um durch geeignete Überwachung der hierzu relevanten physiologischen Parameter einen optimalen Trainingserfolg zu erzielen.

Die Genauigkeit bei der Klassifizierung eines bestimmten physiologischen Zustands lässt sich durch Zusatzsensoren weiter verbessern. Dem liegt insbesondere die Erkenntnis zugrunde, dass zur sicheren Klassifizierung eines Zustands möglichst viele Symptome herangezogen werden. Daher weist das Hörsystem in einer zweckmäßigen Ausgestaltung einen Zusatzsensor auf, der kein Mikrofon ist und ein Zusatzsignal erfasst, welches zusätzlich zum Audiosignal zur Ermittlung und insbesondere auch Klassifizierung des Zustands dient. Ein besonderer Vorteil besteht nun insbesondere darin, dass mittels des Zusatzsensors beispielsweise ein weiteres Symptom eines bestimmten Zustands gemessen wird und dadurch insgesamt eine verbesserte, d.h. insbesondere sicherere Klassifizierung des Zustands gewährleistet ist. So wird beispielsweise ein Sturz anhand der beiden Symptome Aufschlaggeräusch und Lageänderung des Körpers erkannt, wobei die Lageänderung mittels eines Beschleunigungssensors als Zusatzsensor erkannt wird.

In einer geeigneten Variante ist die Steuereinheit ausgebildet, zur Ermittlung des physiologischen Zustands ein Symptom des Zustands redundant zu ermitteln, nämlich auf Grundlage des Zusatzsignals sowie auf Grundlage des Audiosignals. Mit anderen Worten: Anstelle der oder zusätzlich zur Ermittlung zweier unterschiedlicher Symptome eines Zustands mittels Mikrofon und Zusatzsensors erfolgt in dieser Variante die redundante Ermittlung eines einzelnen Symptoms des Zustands mittels zweier Sensoren, nämlich dem Mikrofon und dem Zusatzsensor. Auf diese Weise ist die Erkennung des Symptoms besonders sicher und eine Fehlinterpretation aufgrund der Verwendung lediglich eines Sensors, d.h. eines Mikrofons oder Zusatzsensors, entsprechend reduziert oder sogar vermieden.

Insgesamt ergeben sich somit für einen Zusatzsensor prinzipiell zwei unterschiedliche Verwendungsmöglichkeiten, nämlich zum einen die separate Detektion eines zusätzlichen Symptoms zur Klassifizierung des Zustands und zum anderen die redundante Ermittlung eines vom Mikrofon bereits ermittelten Symptoms.

Der Zusatzsensor ist bevorzugterweise zur Messung einer Messgröße ausgebildet, ausgewählt aus einer Gruppe von Messgrößen, umfassend: Beschleunigung, Temperatur, insbesondere Körpertemperatur, elektrischer Widerstand der Haut optische Eigenschaften der Haut, d.h. insbesondere Transmission, Absorption oder Reflektion, und Blutsauerstoffgehalt. Entsprechend ist der Zusatzsensor also beispielsweise als Beschleunigungssensor, Temperatursensor, Widerstands- oder Leitfähigkeitsmesser, optischer Detektor oder Pulsoximeter ausgebildet. Die Messgröße ist insbesondere ein physiologischer Parameter des Anwenders.

In einer geeigneten Ausführungsform ist der Zusatzsensor als Beschleunigungssensor ausgebildet und ermöglicht so insbesondere die Detektion eines Sturzes. Dabei ist insbesondere bei einem Hörgerät die Verwendung eines Beschleunigungssensors sinnvoller als beispielsweise in einem Armband oder mitgeführten Smartphone, da ein Hörgerät üblicherweise vergleichsweise fest am Kopf des Anwenders getragen wird, wohingegen andere Geräte üblicherweise zusätzlichen Bewegungen ausgesetzt sind, beispielsweise am Handgelenk, mit dem Resultat einer höheren Fehlerquote.

In einer weiteren geeigneten Ausführungsform ist ein Beschleunigungssensor mit einem Mikrofon kombiniert, sodass in redundanter und fehlersicherer Weiser Niesgeräusche des Anwenders erkannt werden. Die Kombination der Auswertungsergebnisse von Zusatzsignal und Audiosignal ermöglicht nun eine besonders sichere Unterscheidung zwischen einem Sturz des Anwenders und einem Niesen des Anwenders, da in ersterem Fall das Mikrofon entsprechend keine Niesgeräusche erfasst. Der Zusatzsensor erfasst also zunächst eine Beschleunigung, die möglicherweise nicht eindeutig einem Sturz oder einem Kopfnicken zuzuordnen ist. Die zusätzliche Auswertung des Audiosignals ermöglicht jedoch eine besonders sichere und eindeutige Zuordnung, sodass entweder ein Niesen oder eine allgemeine Lageänderung des Körpers des Anwenders erkannt wird und somit zuverlässig entweder auf eine Erkältung oder einen Sturz geschlossen wird.

Alternativ oder zusätzlich zu den oben beispielhaft genannten Ausführungsformen lassen sich Audiosignale und Zusatzsignale auch zur verbesserten Erkennung einer Vielzahl weiterer physiologischer Zustände kombinieren, von denen im Folgenden einige aufgeführt sind:
In einer ersten Variante werden vom Mikrofon aufgenommene Atemgeräusche analysiert und mit einer Analyse von Daten eines Beschleunigungssensors kombiniert, um Ruhephasen und Unruhephasen im Schlaf des Anwenders zu erkennen und somit dessen Schlafverhalten zu bewerten.

In einer weiteren Variante wird die physische Aktivität des Anwenders bewertet, indem beispielsweise Trittschall und Pulsschlag über ein Körperschallmikrofon erfasst werden, in Kombination mit durch einen Beschleunigungssensor detektierter körperlicher Bewegung. Dadurch wird dann insgesamt ermittelt, ob der Anwender beispielsweise ein bestimmtes Bewegungspensum absolviert oder es werden Ausdauer und Kondition des Anwenders ermittelt.

In einer weiteren Variante wird ein physiologischer Zustand als Stress klassifiziert, wobei dann als Symptome eine Änderung der Stimmlage, d.h. insbesondere des Frequenzspektrums insgesamt, des Anwenders, ein erhöhter Puls und/oder eine Absenkung der Sauerstoffsättigung ermittelt werden. Besonders in dieser Variante, aber geeigneterweise auch allgemein, erfolgt ein Vergleich der ermittelten physiologischen Parameter mit zuvor im Sinne einer Eichemssung gemessenen Normalwerten, um das Vorliegen eines bestimmten Symptoms zu erkennen. So wird mittels der Steuereinheit beispielsweise in einer Eichmessung zunächst ein Normalpuls bestimmt und gespeichert und im weiteren Betrieb dann ein aktuell gemessener Puls mit dem Normalpuls verglichen. Bei einer bestimmten Abweichung beispielsweise um einen Faktor zwei, wird dann ein erhöhter Puls als Symptom erkannt. In ähnlicher Weise wird alternativ oder zusätzlich eine aktuell gemessene Stimmlage mit einer Normalstimmlage verglichen, um eine entsprechende Abweichung zu ermitteln.

Auch Atemprobleme sind als physiologischer Zustand in einer weiteren Variante erkennbar, indem charakteristische, beispielsweise rasselnde Atemgeräusche oder beschleunigte Atmung mittels des Mikrofons erfasst und mittels der Steuereinheit erkannt werden. Dies wird geeigneterweise mit einer durch einen zusätzlichen Beschleunigungssensor erkannten Unruhe und erhöhten physischen Aktivität des Anwenders kombiniert, um die Atmung des Anwenders zu bewerten. Ein beispielhafter Zustand ist eine respiratorische Insuffizienz, welche anhand der genannten Merkmale klassifiziert wird.

Eine Erkältung wird in einer weiteren Variante durch Erfassung und Erkennung von Nies- und/oder Hustgeräuschen im Frequenzspektrum des Audiosignals ermittelt. Zusätzlich wird in einer Weiterbildung eine insbesondere in Richtung tieferer Frequenzen verschobene Stimmlage gemessen und/oder mit Beschleunigungssensoren werden Kopfbewegungen erkannt, welche für Niesen und Husten charakteristisch sind. Weiterhin wird zweckmäßigerweise mittels eines Temperatursensors die Körpertemperatur des Anwenders ermittelt und eine erhöhte Temperatur als Symptom erkannt und zur Klassifizierung eines physiologischen Zustands als Erkältung verwendet.

Durch Analyse der Redezeit des Anwenders, besonders in Kombination mit der Erkennung anderer Stimmen als derjenigen des Anwenders, schließt das Hörsystem in einer weiteren Variante auf die soziale Aktivität des Anwenders. Beispielsweise wird dadurch eine verminderte soziale Aktivität als Zustand erkannt. Als Merkmal zur Klassifizierung dient beispielsweise die Unterschreitung einer bestimmten täglichen Gesprächszeit, d.h. der Kommunikation mit einem anderen Menschen.

Um insbesondere ein Fehlverhalten eines Sensors, nämlich eines Mikrofons oder eines Zusatzsensors, besonders effektiv zu erkennen, weist das Hörsystem in einer zweckmäßigen Ausgestaltung einen weiteren Sensor gleicher Art auf. Der entsprechende Sensor ist somit am Hörsystem redundant ausgebildet, und ein Fehlverhalten eines der Sensoren ist durch Vergleich der von den beiden Sensoren gelieferten Signale erkennbar. Dabei liefern die beiden redundanten Sensoren im Idealfall gleiche Signale, insbesondere innerhalb eines vorgegebenen Toleranzbereichs, und eine Abweichung wird von der Steuereinheit als Fehlverhalten eines der Sensoren erkannt. Bei Erkennung eines entsprechenden Fehlverhaltens wird dann beispielsweise der Anwender mittels der Steuereinheit auf das Fehlverhalten des entsprechenden Sensors hingewiesen. Im weiteren Betrieb werden die entsprechenden Sensorsignale zweckmäßigerweise nicht weiter verwendet.

Die beiden gleichartigen Zusatzsensoren weisen jeweils eine Messungenauigkeit auf, wobei in einer zweckmäßigen Ausgestaltung die Steuereinheit ausgebildet ist, die Zusatzsignale zu verwerfen, falls eine Abweichung der Zusatzsignale der beiden Zusatzsensoren größer ist als die jeweilige Messungenauigkeit. Die beiden gleichartigen, d.h. redundanten Zusatzsensoren werden somit insbesondere regelmäßig von der Steuereinheit auf ein Fehlverhalten untersucht, indem die Zusatzsignale, beispielsweise deren Amplitude, gegeneinander verglichen werden, um eine Signaldifferenz abzuleiten, welche dann mit der Messungenauigkeit verglichen wird. Überschreitet die Signaldifferenz die Messungenauigkeit, wird auf ein Fehlverhalten des Zusatzsensors geschlossen.

In einer bevorzugten Ausgestaltung sind die beiden Sensoren als Zusatzsensoren ausgebildet und jeweils in einem der beiden Hörgeräte angeordnet, zur Messung eines binauralen Zusatzsignals, wobei die beiden Zusatzsensoren jeweils insbesondere als Beschleunigungssensor ausgebildet sind. Dieser Ausgestaltung liegt der Gedanke zugrunde, dass die sich für die Mikrofone aus der binauralen Verrechnung ergebenden Vorteile in ähnlicher Weise auch für Zusatzsensoren verwendbar sind. Dies ist besonders bei Beschleunigungssensoren der Fall, bei denen dann mittels binauraler Verrechnung der Zusatzsignale die genaue Bewegungsrichtung des Anwenders, insbesondere dessen Kopf, gemessen wird. Durch die binaurale Verrechnung ergibt sich dann ein vorteilhafter Informationsmehrwert im Vergleich zur lediglich separaten Auswertung der Zusatzsensoren. Beispielsweise unterscheidet die Steuereinheit dann aufgrund des binauralen Zusatzsignals zwischen einer Vorwärtsbewegung oder einer Drehbewegung des Kopfes des Anwenders, d.h. zwischen einer translatorischen und einer rotatorischen Bewegung wodurch eine entsprechend verbesserte Klassifizierung von Symptomen und Zuständen möglich ist. Wird dann beispielsweise eine Vorwärtsbewegung erkannt, wird auf ein Niesen geschlossen. Die Häufigkeit von Drehbewegungen wird in einer Variante als Indikator für körperliche Aktivität allgemein oder als Anzeichen eines unruhigen Schlafes verwendet. In einer weiteren Variante werden als Zusatzsensoren Bewegungssensoren verwendet, die vorzugsweise auf unterschiedlichen Seiten des Kopfes des Anwenders positioniert sind. Auch in dieser Ausgestaltung ist es zweckmäßig, die Zusatzsignale beider Seiten zu vergleichen und bei einer starken Abweichung zu verwerfen. Registriert beispielsweise lediglich einer der Bewegungssensoren eine hinreichend starke Beschleunigung, die z.B. durch einen freien Fall verursacht ist, wird darauf geschlossen, dass das Hörgerät mit ebendiesem Bewegungssensor herunterfällt. Registrieren hingegen die Bewegungssensoren beider Hörgeräte einen freien Fall, wird auf einen Sturz des Anwenders geschlossen.

Prinzipiell ist die Steuereinheit in einer geeigneten Ausgestaltung in das Hörgerät oder die Hörgeräte integriert. In einer ebenfalls geeigneten Variante ist die Steuereinheit dagegen außerhalb des Hörgeräts angeordnet, beispielsweise als Teil eines Computers oder Smartphones. Das Hörgerät weist dann zweckmäßigerweise eine Vorverarbeitungseinheit auf, zur Bearbeitung des Zusatzsignals und/oder des Audiosignals vor einer Weitergabe an die Steuereinheit. Auf diese Weise erfolgt dann im Hörgerät quasi eine Vorauswahl der an die Steuereinheit zu übertragenden Signale, beispielsweise basierend auf Schwellwerten oder Änderungen des Signals über Zeit. Durch diese Vorauswahl ergibt sich insbesondere der Vorteil, dass eine zwischen dem Hörgerät und der Steuereinheit ausgebildete Datenverbindung entsprechend geschont wird, da lediglich die vorausgewählten Signale übertragen werden, wodurch dann entsprechend der Bandbreite und auch Energie zum Betrieb der Datenverbindung eingespart werden. Dies ist insbesondere hinsichtlich der häufig eingeschränkten Energieversorgung des Hörgeräts mittels einer Batterie von besonderem Vorteil.

Grundsätzlich ist bei einem binauralen Hörsystem die Verwendung von zwei, insbesondere miteinander kommunizierender Steuereinheiten denkbar. Vorteilhafterweise erfolgt jedoch eine Konsolidierung derart, dass eine gemeinsame Steuereinheit angeordnet ist, an welche sämtliche Sensordaten weitergegeben werden.

Geeigneterweise weist die Steuereinheit eine Verstärkereinheit auf, die mit einem Hörer, d.h. einem Lautsprecher des Hörgeräts, verbunden ist, zur Ausgabe des Audiosignals, sowie eine Analyseeinheit, zur Analyse des Audiosignals und zur Klassifizierung des physiologischen Zustands. Dazu umfasst die Analyseeinheit insbesondere einen Klassifikator ausgebildet. Die Steuereinheit umfasst somit zwei Teileinheiten, nämlich die Verstärkereinheit und die Analyseeinheit, welche entsprechend getrennt die jeweiligen Aufgaben ausführen. Das von einem jeweiligen Mikrofon erfasste Audiosignal wird dann zunächst im Betrieb an die Steuereinheit weitergegeben, und dann jeweils an die beiden Teileinheiten weitergereicht, zur separaten Verarbeitung. Ein besonderer Vorteil dieser Ausgestaltung ist dann insbesondere, dass die Steuereinheit sozusagen spezialisierte Teileinheiten aufweist, einmal zur Realisierung der Grundfunktion des Hörsystems und einmal zur Realisierung der Zusatzfunktion.

Die Analyseeinheit ist selbst zweckmäßigerweise in mehrere Untereinheiten unterteilt, nämlich insbesondere in eine Audiosignalverarbeitungseinheit, zur Auswertung und Analyse von Audiosignalen, und eine Zusatzsignalverarbeitungseinheit, zur Auswertung und Analyse von Zusatzsignalen. Bei einem Hörsystem, welches keine Zusatzsensoren aufweist, ist lediglich eine Audiosignalverarbeitungseinheit angeordnet.

Die Audiosignalverarbeitungseinheit ist insbesondere derart ausgebildet, dass die eingehenden Audiosignale hinsichtlich bestimmter, vorgegebener Merkmale untersucht werden, diese Merkmale erkannt werden und auf Grundlage der erkannten Merkmale eine Klassifizierung des vom Mikrofon erfassten Geräuschs als ein bestimmtes Symptom erfolgt. Die Merkmale sind hierbei beispielsweise bestimmte Amplitudenverläufe im Frequenzspektrum, eine bestimmte Amplitude in einem vorgegebenen Frequenzband oder eine zeitliche Veränderung einer Amplitude in einem bestimmten Frequenzband.

Ähnlich der Audiosignalverarbeitungseinheit ist die Zusatzsignalverarbeitungseinheit insbesondere derart ausgebildet, dass die eingehenden Zusatzsignale hinsichtlich bestimmter, vorgegebener Merkmale untersucht werden, diese Merkmale erkannt werden und auf Grundlage der erkannten Merkmale eine Klassifizierung des vom Zusatzsensors erfassten physiologischen Parameters als ein bestimmtes Symptom erfolgt. Die Merkmale sind hierbei beispielsweise eine bestimmte, zum Beispiel einen vorgegebenen Schwellwert überschreitende Amplitude des Zusatzsignals oder ein bestimmter zeitlicher Verlauf der Amplitude.

Zur Übertragung der verschiedenen Sensorsignale zur Steuereinheit und/oder zur Übertragung von Daten zwischen verschiedenen Teileinheiten und/oder Untereinheiten der Steuereinheit, weist das Hörsystem eine Anzahl von Datenverbindungen auf, die grundsätzlich jeweils entweder drahtgebunden oder drahtlos ausgebildet sind. Bevorzugterweise sind zumindest diejenigen Datenverbindungen drahtlos ausgeführt, welche zur Datenübertragung zwischen zwei Hörgeräten oder zwischen einem Hörgerät und einem externen Gerät dienen.

Vorzugsweise ist die Verstärkereinheit in das Hörgerät integriert und die Analyseeinheit ist außerhalb des Hörgeräts angeordnet. Bei einem binauralen Hörgerät ist insbesondere in jedes der Hörgeräte eine Verstärkereinheit integriert. Mit anderen Worten: Die Steuereinheit ist in funktionelle Teileinheiten unterteilt, welche einerseits intern und andererseits extern bezüglich des Hörgeräts oder der Hörgeräte angeordnet sind. Durch diese getrennte Ausführung der Steuereinheit erfolgt ein besonders effizientes Energiemanagement sowie eine Schonung der Datenrate einer möglichen Datenverbindung zwischen den Hörgeräten. Die vergleichsweise energie- und datenintensive Klassifizierung von physiologischen Zuständen des Anwenders erfolgt dann vorteilhaft extern, d.h. auf einem externen Gerät, wohingegen die insbesondere durchgehend benötigte Grundfunktionalität der Verstärkung von Umgebungsgeräuschen intern, d.h. im Hörgerät erfolgt. Da an das externe Gerät üblicherweise weniger strikte Anforderungen hinsichtlich dessen Dimensionierung gelten, als für die Hörgeräte, ist das externe Gerät zweckmäßigerweise mit einer deutlich leistungsfähigeren Energieversorgung und Rechenkapazität ausgestattet.

In einer Variante, bei welcher kein externes Gerät vorhanden ist und die Steuereinheit vollständig im Hörgerät untergebracht ist oder auf zwei Hörgeräte aufgeteilt ist, lässt sich die Zusatzfunktionalität zweckmäßigerweise abschalten, ohne dass die Grundfunktionalität davon beeinträchtigt wird. Beispielsweise wird dann im Falle einer fast leeren Batterie des Hörgeräts die Datenverbindung zur Analyseeinheit vom Hörsystem automatisch ausgeschaltet, um auf diese Weise Energie zu sparen und im Sinne eines Energiesparmodus über einen längeren Zeitraum zumindest noch die Grundfunktion bereitstellen zu können.

Bei einem Verfahren zum Betrieb eines binauralen Hörsystems, insbesondere wie oben beschrieben, mit zwei Hörgeräten, wird mittels eines jeweiligen Mikrofons eines jeweiligen der Hörgeräte ein jeweiliges Audiosignal erfasst und die Audiosignale werden an eine Steuereinheit weitergegeben. D.h. insbesondere, dass jedes der Hörgeräte ein Mikrofon aufweist, mittels welchem ein Audiosignal erfasst wird. Mittels der Steuereinheit und auf Grundlage der Audiosignale wird ein physiologischer Zustand des Anwenders klassifiziert. Zusätzlich wird mittels der Steuereinheit auf Grundlage der Audiosignale ein Eigenanteil, der vom Anwender stammt, hervorgehoben und zur Klassifizierung des physiologischen Zustands verwendet.

In einer zweckmäßigen Ausgestaltung des Verfahrens wird der physiologische Zustand als ein Zustand aus einer Gruppe von insbesondere nicht-pathologischen Zuständen klassifiziert, umfassend: Sturz, Schlafverhalten, soziale Aktivität, Bewegungsaktivität und Stresslevel. Der besondere Vorteil dieser Variante besteht insbesondere darin, dass durch das Hörsystem eine generelle Überwachung und Bewertung der körperlichen Leistungsfähigkeit des Anwenders erfolgt. Dabei wird insbesondere auf die Erkennung und Diagnose von Krankheiten verzichtet. Vielmehr dient das Hörsystem zusätzlich zur Grundfunktionalität zur Eigenüberwachung oder -analyse durch den Anwender, im Sinne einer Leistungsüberwachung.

So lässt sich das Hörsystem dann beispielsweise bei sportlicher Aktivität auch als Leistungsmesser einsetzen, ohne dass der Anwender ein zusätzliches hierzu speziell ausgebildetes Gerät zu tragen braucht. Die körpernahe Unterbringung des Hörgeräts, besonders die Anordnung am Kopf, hat hier besondere Vorteile gegenüber anderen Stellen am Körper. So ist der Kopfbereich und insbesondere der Gehörgang besonders zur Messung vom Körperschall und Anwendergeräuschen geeignet.

Nachfolgend werden Ausführungsbeispiele der Erfindung anhand einer Zeichnung näher erläutert. Darin zeigen jeweils schematisch:
- Fig. 1: ein binaurales Hörsystem,
- Fig. 2: ein weiteres binaurales Hörsystem,
- Fig. 3: ein weiteres binaurales Hörsystem mit zwei Hörgeräten und einem externen Gerät, und
- Fig. 4: schematisch die Klassifizierung eines physiologischen Zustands anhand von Symptomen.

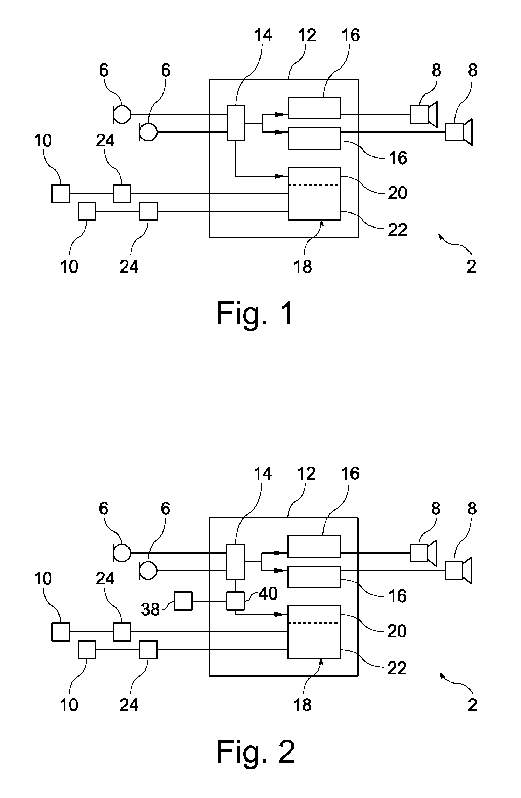

In Fig. 1 ist schematisch eine erste Variante eines Hörsystems 2 dargestellt. Dieses ist insbesondere ein binaurales Hörsystem 2, mit zwei nicht näher dargestellten Hörgeräten 4, welche hier jeweils ein Mikrofon 6, einen Hörer 8 sowie einen Zusatzsensor 10 umfassen. Das Hörsystem 2 weist eine Grundfunktionalität auf, bei welcher mittels der Mikrofone 6 Geräusche als Audiosignale erfasst werden und zur Weiterverarbeitung zunächst an eine Steuereinheit 12 weitergegeben werden. Die Audiosignale werden dabei zunächst einer binauralen Verrechnungseinheit 14 zugeführt, welche ein Teil der Steuereinheit 12 ist.

Die binaurale Verrechnungseinheit 14 wird einerseits in herkömmlicher Weise verwendet, nämlich derart, dass diese Audiosignale hervorhebt, welche zumeist aus der Umgebung des Anwenders kommen. Je nach Hörsituation kann diese Verrechnungseinheit 14 dann Schallquellen aus allen Richtungen gleichermaßen hervorheben oder aber gezielt Schallquellen, welche sich in einem breiten oder schmalen Richtungswinkelbereich befinden. Die binaurale Verrechnungseinheit 14 erzeugt also Audiosignale, die entsprechend der Hörsituation die für den Anwender relevanten Schallquellen der Umgebung optimal hervorheben und die dann über den jeweiligen Hörer 8 ausgegeben werden. Um dabei die Geräusche für den Anwender, welcher möglicherweise eine Hörschädigung hat, hörbar zu machen, ist den Hörern 8 jeweils eine Verstärkereinheit 16 zugeordnet, welche diese Audiosignale geeignet verstärken.

Zusätzlich werden mittels der Mikrofone 6 insbesondere auch Geräusche aus einem schmalen und bezüglich des Anwenders nach vorn gerichteten Winkelbereich aufgenommen. Die binaurale Verrechnungseinheit 14 wird dann zusätzlich zur herkömmlichen Verwendung auch derart verwendet, dass diese Anwendergeräusche ermittelt und hervorhebt, beispielsweise Geräusche, die vom Gesicht oder vom Kehlkopf des Anwenders ausgehen und deshalb konstant aus ebendiesem Winkelbereich von den Mikrofonen 6 aufgenommen werden. Damit ist eine Vielzahl an Geräusch umfasst, darunter: Niesen, Husten, Räuspern, Atmung und die Stimme des Anwenders. Die auf diese Weise binaural verrechneten Audiosignale werden dann für eine Zusatzfunktionalität des Hörsystems 2 genutzt, bei welcher die Audiosignale der Mikrofone 6 zusätzlich zur Klassifizierung eines physiologischen Zustands Z des Anwenders verwendet werden. Dazu weist die Steuereinheit 12 eine Analyseeinheit 18 auf, welcher diverse Sensorsignale zugeführt werden, auf deren Grundlage dann eine Anzahl von Symptomen S des Zustands Z ermittelt werden, um den Zustand Z zu klassifizieren. In dem hier gezeigten Ausführungsbeispiel weist die Analyseeinheit 18 dazu einerseits eine Audiosignalverarbeitungseinheit 20 sowie eine Zusatzsignalverarbeitungseinheit 22 auf. Die Audiosignalverarbeitungseinheit 20 dient dabei der Analyse und Bewertung der von den Mikrofonen 6 aufgenommenen Audiosignale und deren Zuordnung zu bestimmten Symptomen S. In dem in Fig. 1 gezeigten Ausführungsbeispiel werden der Audiosignalverarbeitungseinheit 20 dazu Signale der binauralen Verrechnungseinheit 14 zugeführt. Diese unterscheiden sich von jenen den Verstärkereinheiten 16 zugeführten Audiosignalen insbesondere dadurch, dass zwecks Analyse vorrangig die Eigenanteile der Audiosignale ausgewählt und an die Analyseeinheit 18 weitergegeben werden. Diese Eigenanteile und Anwendergeräusche sind im Vergleich zu Umgebungsgeräuschen für die Klassifizierung eines physiologischen Zustands Z von vorrangiger Bedeutung, da letztere üblicherweise keinen Schluss auf den Zustand Z des Anwenders zulassen.

Um die Klassifizierung des Zustands Z sowie die Erkennung und Bewertung der Symptome S weiter zu verbessern, werden der Analyseeinheit 18 weiterhin Zusatzsignale von Zusatzsensoren 10 zugeführt. Diese Zusatzsignale werden dabei insbesondere der Zusatzsignalverarbeitungseinheit 22 der Analyseeinheit 18 zugeführt und dort entsprechend interpretiert und zur Klassifizierung von Symptomen S verwendet.

Um insbesondere die Menge an von einem jeweiligen Hörgerät 4 an die Steuereinheit 12 übertragenen Sensorsignale, d.h. insbesondere die Menge an Daten, zu reduzieren, weist jedes der Hörgeräte 4 zusätzlich eine Vorverarbeitungseinheit 24 auf, welche vorrangig der Auswahl derjenigen Zusatzsignale dient, die tatsächlich an die Steuereinheit 12 übertragen werden. Nicht relevante Signale werden dann durch die Vorverarbeitungseinheit 24 herausgefiltert und belasten dann die Datenverbindung nicht.

Fig. 2 zeigt eine alternative Ausgestaltung des Hörsystems 2, welches auch binaural ausgestaltet ist, zusätzlich jedoch ein Körperschallmikrofon 38 und eine Audiosignalkombinationseinheit 40 aufweist. Diese kombiniert das Audiosignal der binauralen Verrechnungseinheit 14 mit dem Audiosignal des Körperschallmikrofons 38, wodurch die Anwendergeräusche noch einmal gegenüber anderen Geräuschen hervorgehoben werden. Dazu werden die beiden unterschiedlichen Audiosignale in der Audiosignalkombinationseinheit 40 beispielsweise addiert. Die Hervorhebung der Anwendergeräusche basiert dabei wesentlich auf der Tatsache, dass das Körperschallmikrofon 38 vorrangig Anwendergeräusche aufnimmt und kaum Umgebungsgeräusche, sodass eine Kombination mit dem Audiosignal der binauralen Verrechnungseinheit 14, welches insbesondere sowohl Anwendergeräusche als auch Umgebungsgeräusche enthält, zu einer relativen Verstärkung der Anwendergeräusche führt. Die Audiosignalkombinationseinheit 40 ist in einer nicht gezeigten Variante als Teil der Analyseeinheit 18 ausgebildet. Generell ist die Audiosignalkombinationseinheit 40 der Audiosignalverarbeitungseinheit 20 vorgeschaltet, sodass letzterer das aus der Kombination resultierende Audiosignal mit hervorgehobenen Anwendergeräuschen zur verbesserten Analyse zur Verfügung steht.

Zusätzlich oder alternativ erfolgt auch eine binaurale Verrechnung der Zusatzsignale der Zusatzsensoren 10. Dies ist insbesondere bei Beschleunigungssensoren als Zusatzsensoren 10 von Bedeutung, da hier die Verrechnung von Zusatzsignalen von vorzugsweise binaural angeordneten Beschleunigungssensoren einen Informationsmehrwert schafft, indem die Richtung der Beschleunigung in verbesserter Weise messbar ist. Desweiteren sind dadurch insbesondere auch Verschiebungsbewegungen, die z.B. bei einem Kopfnicken auftreten, besser von Rotationsbewegungen, also einer Drehung des Kopfes, unterscheidbar. Entsprechend ist dann beispielsweise eine weitere binaurale Verrechnungseinheit für die Zusatzsignale in der Steuereinheit 12 vorhanden oder direkt in die Signalverarbeitungseinheit 22 integriert.

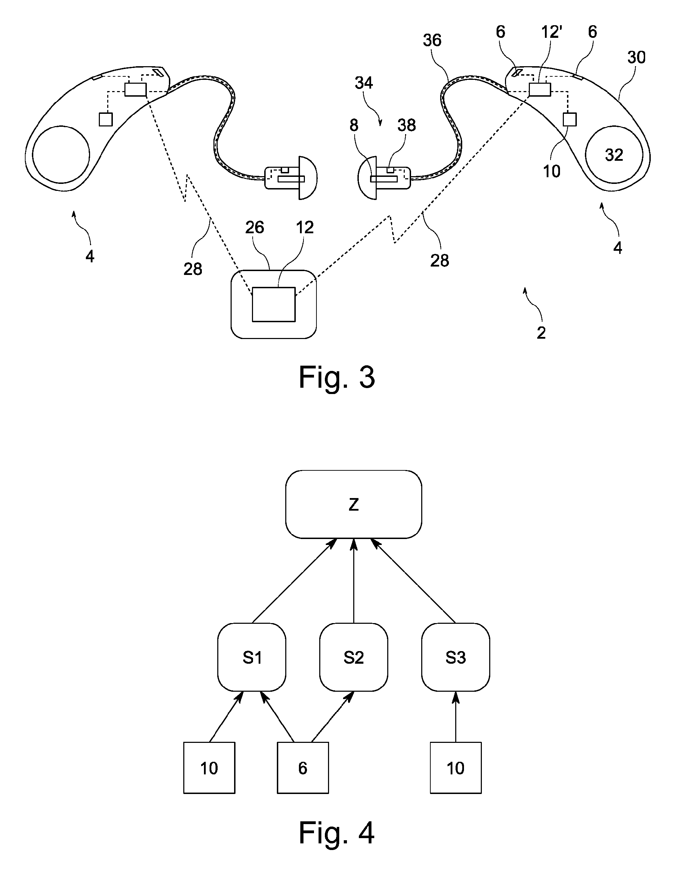

Hinsichtlich der genauen Anordnung und Ausgestaltung der Steuereinheit 12 sind diverse Ausführungen des Hörsystems 2 denkbar. Fig. 3 zeigt hierzu ein Ausführungsbeispiel, bei welchem die Steuereinheit 12 extern ausgebildet und in einem externen Gerät 26 angeordnet ist, beispielsweise einem Smartphone oder einem Computer. Die Hörgeräte 4 des binauralen Hörsystems 2 weisen dann jeweils eine Datenverbindung 28 mit einer im jeweiligen im Hörgerät 4 untergebrachten Übertragungseinheit auf, mittels welcher die Audiosignale und die Zusatzsignale, insbesondere die vorverarbeiteten Zusatzsignale an die Steuereinheit 12 übertragen werden. Die Datenverbindung 28 ist hier eine Drahtlosverbindung. In dem gezeigten Ausführungsbeispiel sind die beiden Hörgeräte 4 zumindest funktionell gleichartig ausgebildet.

Zweckmäßigerweise sind zumindest die Verstärkereinheiten 16 in den Hörgeräten 4 untergebracht und nicht im externen Gerät 26. Die Verstärkung erfolgt dann insbesondere lokal im jeweiligen Hörgerät. In Fig. 3 sind die in den Hörgeräten 4 untergebrachten Teile der Steuereinheit 12 als lokale Steuereinheiten 12' dargestellt. in dem gezeigten Ausführungsbeispiel umfassen die lokalen Steuereinheiten 12' jeweils zumindest eine Verstärkereinheit 16 sowie eine Übertragungseinheit für die Datenverbindung 28. Die binaurale Verrechnung sowie die Analyse der Audiosignale und Zusatzsignale, d.h. allgemein der Sensorsignale, erfolgt dagegen im externen Gerät 26. Dieses umfasst insbesondere eine nicht näher bezeichnete separate Energieversorgung, beispielsweise ein Batterie. In einer alternativen Ausgestaltung sind auch weitere Teile der Steuereinheit 12 in die Hörgeräte 4 integriert. So ist beispielsweise denkbar, dass die Steuereinheit 12 vollständig in eines der Hörgeräte 4 integriert ist, und dann in geeigneter Weise eine Datenverbindung 28 mit dem anderen Hörgerät 4 ausgebildet ist. Alternativ ist auch denkbar, dass jedes der Hörgeräte 4 jeweils eine Steuereinheit 12 umfasst, wobei die beiden Steuereinheiten 12 dann miteinander über eine geeignete Datenverbindung 28 kommunizieren.

Die in Fig. 3 gezeigten Hörgeräte 4 sind als sogenannte BTE-Geräte ausgeführt, d.h. weisen jeweils ein Gehäuse 30 auf, in welchem die wesentlichen Komponenten angeordnet sind. Dabei ist eines der Hörgeräte 4 als linkes Hörgerät 4 ausgebildet, das andere dann als rechtes Hörgerät 4. Insbesondere weist ein jeweiliges Hörgerät 4 in Fig. 3 zwei Mikrofone 6 auf, sowie eine Batterie 32 und ein Ohrstück 34, welches vom Anwender im Gehörgang getragen wird. Dagegen wird das Gehäuse 30 vom Anwender hinter dem Ohr getragen. Das Ohrstück 34 ist mit dem Gehäuse 30 über ein Verbindungsstück 36 verbunden.

Je nach Ausgestaltung des Hörgeräts 4 ist entweder, wie in Fig. 3 gezeigt, im Ohrstück 34 ein Hörer 8 untergebracht, welcher dann über ein nicht näher dargestelltes Kabel mit dem Gehäuse 30 und insbesondere der lokalen Steuereinheit 12' verbunden ist. In einer nicht gezeigten Alternative ist der Hörer 8 jedoch im Gehäuse 30 untergebracht und das Verbindungsstück 36 ist dann als Schallschlauch ausgebildet, zur Übertragung von Schall in den Gehörgang.

Das hier gezeigte Hörgerät 4 weist weiterhin ein Körperschallmikrofon 38 auf, welches ein Teil des Ohrstücks 34 ist und daher im getragenen Zustand im Gehörgang des Anwenders sitzt. Dadurch ist das Körperschallmikrofon 38 besonders zur Erfassung von Anwendergeräuschen geeignet, während Umgebungsgeräusche vom Körperschallmikrofon 38 gar nicht oder lediglich in vermindertem Maße aufgenommen werden. Das Körperschallmikrofon 38 ist hier insbesondere als separates Mikrofon 6 ausgeführt und dient insbesondere gleichzeitig als Zusatzsensor 10. Die Analyse der vom Körperschallmikrofon 38 erfassten Audiosignale erfolgt dabei in der Audiosignalverarbeitungseinheit 20. In einer nicht gezeigten Alternative wird eines der regulären Mikrofone 6 als Körperschallmikrofon 38 verwendet und dient so auch gleichermaßen als Zusatzsensor 10, dessen Zusatzsignale jedoch sinnvollerweise mittels der Audiosignalverarbeitungseinheit 20 analysiert werden.

Die in Fig. 3 gezeigten Hörgeräte 4 weisen zusätzlich jeweils einen Zusatzsensor 10 auf, der hier insbesondere als Beschleunigungssensor ausgebildet ist. Der Zusatzsensor 10 ist dabei im jeweiligen Gehäuse 30 untergebracht. In einer Variante sind weitere und/oder andere Zusatzsensoren 10 im oder am Hörgerät angeordnet. Dabei ist auch eine Unterbringung an oder in dem Ohrstück 38 oder dem Verbindungsstück 36 denkbar.

Anhand von Fig. 4 wird im Folgenden die Klassifizierung eines physiologischen Zustands Z des Anwenders erläutert. Dabei wird beispielhaft die Klassifizierung des physiologischen Zustands Z als Erkältung beschrieben. Der Zustand Z ist Ursache einer Anzahl von Symptomen S1, S2, S3. Im Falle einer Erkältung entspricht das Symptom S1 zum Beispiel einem Niesen, das Symptom S2 einer veränderten Stimmlage des Anwenders beim Sprechen und das Symptom S3 einer von einem Normalzustand abweichenden Körpertemperatur. Diese Symptome S1, S2, S3 werden nun als Merkmale einer Erkältung mittels Analyse und Bewertung der Audiosignale und Zusatzsignale ermittelt und gegebenenfalls auch quantifiziert, um anschließend den Zustand Z zu erkennen und als Erkältung zu klassifizieren.

Das Symptom S1 wird dabei auf besonders sichere Weise redundant sowohl durch einen als Beschleunigungssensor ausgebildeten Zusatzsensor 10 ermittelt als auch durch Analyse der von den Mikrofonen 6 stammenden Audiosignale. Die Erkennung eines Niesens alleinig auf Grundlage von Beschleunigungssensoren ist potenziell stark fehlerbehaftet, da beispielsweise auch ein Sturz oder auch ein einfaches Kopfnicken von den Zusatzsensoren 10 entsprechend registriert würde und dann fälschlicherweise das Vorliegen eines Niesens gefolgert würde. Zur zusätzlichen Sicherheit bei der Erkennung des Symptoms S1 werden die Audiosignale der Mikrofone 6 hinzugezogen. Die Audiosignale werden dabei in der Audiosignalverarbeitungseinheit 20 auf bestimmte charakteristische Frequenzspektren untersucht, welche einem Niesen zugeordnet werden, sodass also das dem Audiosignal zugrundeliegende Geräusch als Niesen klassifiziert wird. Auch die Erkennung eines Niesens alleinig aufgrund einer Analyse des Frequenzspektrums der Audiosignale ist unter Umständen fehlerbehaftet, in Kombination mit der Auswertung der Zusatzsignale ist jedoch eine zuverlässige Erkennung des Symptoms S1 als Niesen gewährleistet.

Auf Grundlage des Symptoms S1 könnte bereits der Zustand Z als Erkältung klassifiziert werden. Allerdings ist es auch denkbar, dass der Anwender in Situationen niest, in denen keine Erkältung vorliegt. Daher werden die Audiosignale in der Audiosignalverarbeitungseinheit 20 zusätzlich auf eine veränderte Stimmlage als Symptom S2 untersucht. Wird eine solche veränderte und für eine Erkältung charakteristische Verschiebung der Stimmlage erkannt, wird in Kombination mit einer Erkennung des Symptoms S1 mit erhöhter Sicherheit auf eine Erkältung als physiologischen Zustand Z geschlossen.

Zusätzlich ist in Fig. 4 ein weiteres Symptom S3 gezeigt, welches auf einfache Weise mittels eines einfachen Zusatzsensors 10 ermittelt wird, der hier ein Temperatursensor ist, zur Messung der Körpertemperatur des Anwenders. Überschreitet die Körpertemperatur dann einen bestimmten vorgegebenen Grenzwert, so wird auch das Symptom S3 als vorliegend gewertet, wodurch weiterhin eine fehlerfreie Klassifizierung des Zustands Z gewährleistet ist.

Bei der Auswertung der Audiosignale der Mikrofone 6 ist insbesondere die binaurale Verrechnung beim binaural ausgebildeten Hörsystem 2 vorteilhaft. Diese ermöglicht eine sicherere Erkennung von Anwendergeräuschen. Beispielsweise ist es bei der Erkennung des Niesens dann möglich zu unterscheiden, ob ein erkanntes Niesgeräusch überhaupt vom Anwender selbst stammt oder nicht vielmehr von einer anderen Person aus der Umgebung des Anwenders. Die Unterscheidung zwischen Anwendergeräuschen und Umgebungsgeräuschen und insbesondere die vorrangige Verwendung von Anwendergeräuschen zur Klassifizierung des Zustands Z, reduziert dann weiterhin die Fehlerquote bei dieser Klassifizierung.

In einem nicht gezeigten Beispiel wird als physiologischer Zustand Z ein Sturz des Anwenders erkannt, in dem über als Beschleunigungssensoren ausgebildete Zusatzsensoren 10 eine Lageänderung des Körpers des Anwenders als ein Symptom S1, S2, S3 erkannt wird und mittels der Mikrofone 6 ein Aufprallgeräusch als weiteres Symptom S1, S2, S3 detektiert wird. In der Kombination wird dann ein Sturz besonders sicher detektiert und kann beispielsweise auch aufgrund der zusätzlichen Analyse der Audiosignale auch von einem Niesen oder einem Kopfschütteln oder einer gewollten horizontalen Lage beispielsweise beim Schlafen, unterschieden werden.

Insbesondere bei der Erkennung eines Sturzes profitieren sowohl die Zusatzsensoren 10 als auch die Mikrofone 6 jeweils von einer binauralen Verrechnung. So lässt sich aufgrund einer binauralen Verrechnung der Audiosignale der beiden Hörgeräte 4 ein Aufprallgeräusch besonders fehlerfrei als Aufprallgeräusch des Anwenders identifizieren und durch binaurale Verrechnung der Zusatzsignale die genaue Neigung des Anwenders als Fall interpretieren.

Die Erkennung einer Erkältung oder eines Sturzes stellen lediglich zwei von einer Vielzahl von Beispielen zur Klassifizierung eines Zustands Z dar. Allgemein ist es durch Verwendung entsprechend geeignet ausgewählter Zusatzsensoren 10 sowie entsprechender Auswertung und Analyse der Audiosignale in der Audiosignalverarbeitungseinheit 20 möglich, eine Vielzahl von Symptomen S1, S2, S3 zu erkennen und zu bewerten und daraus in besonders sicherer Weise Rückschlüsse auf das Vorliegen eines bestimmten physiologischen Zustands Z zurückzuführen. So ist es beispielsweise auch möglich, das Schlafverhalten des Anwenders als physiologischen Zustand Z zu untersuchen und dabei die Audiosignale beispielsweise hinsichtlich der Atemgeräusche des Anwenders zu analysieren und dabei beispielsweise dessen Atemfrequenz zu bestimmen. In Kombination mit einer Auswertung von Beschleunigungssensoren lassen sich dann besonders effizient Ruhephasen und Unruhephasen während des Schlafens aufzeichnen und hinterher entsprechend auswerten. Auch Atembeschwerden oder insbesondere nichtpathologische Zustände, wie beispielsweise die soziale Aktivität des Anwenders oder dessen physische, insbesondere sportliche Aktivität werden durch entsprechende Kombination geeigneter Zusatzsensoren 10 und Auswertung der Audiosignale der Mikrofone 6 zuverlässig erkannt.

Generell werden die auf diese Weise erkannten Zustände Z und die im Zusammenhang damit gesammelten Daten dann beispielsweise gespeichert und dem Anwender zur Leistungskontrolle oder Gesundheitsüberwachung zur Verfügung gestellt. Alternativ ist es auch denkbar, insbesondere die gesundheitsrelevanten Daten zu speichern und bei einem Arztbesuch einem Arzt zur Analyse und unter Umständen auch Diagnose zur Verfügung zu stellen.

### Bezugszeichenliste

- 2: Hörsystem
- 4: Hörgerät
- 6: Mikrofon
- 8: Hörer
- 10: Zusatzsensor
- 12: Steuereinheit
- 12': lokale Steuereinheit
- 14: binaurale Verrechnungseinheit
- 16: Verstärkereinheit
- 18: Analyseeinheit
- 20: Audiosignalverarbeitungseinheit
- 22: Zusatzsignalverarbeitungseinheit
- 24: Vorverarbeitungseinheit
- 26: externes Gerät
- 28: Datenverbindung
- 30: Gehäuse
- 32: Batterie
- 34: Ohrstück
- 36: Verbindungsstück
- 38: Körperschallmikrofon
- 40: Audiosignalkombinationseinheit

- S1,S2,S3: Symptom
- Z: physiologischer Zustand

## Patentansprüche

1. Hörsystem (2) für einen Anwender, welches binaural ausgebildet ist, mit zwei Hörgeräten (4), insbesondere BTE-Geräten, welche jeweils ein Mikrofon (6) aufweisen, zur Erfassung eines Audiosignals, und mit einer Steuereinheit (12), die ausgebildet ist, auf Grundlage eines Audiosignals einen physiologischen Zustand (Z) des Anwenders zu klassifizieren und auf Grundlage der beiden Audiosignale einen Eigenanteil, der vom Anwender stammt, hervorzuheben und zur Klassifizierung des physiologischen Zustands (Z) zu verwenden.

2. Hörsystem (2) nach einem der vorhergehenden Ansprüche,
**dadurch gekennzeichnet,**
**dass** dieses ein Körperschallmikrofon (38) aufweist, zur Erfassung eines Anwendergeräuschs als Audiosignal, das vom Anwender verursacht ist und insbesondere einer Gruppe von Geräuschen angehört, umfassend: Atemgeräusch, Trittschall, Körperschall, Puls und Stimme.

3. Hörsystem (2) nach dem vorhergehenden Anspruch,
**dadurch gekennzeichnet,**
**dass** die Steuereinheit (12) eine Audiosignalkombinationseinheit (40) aufweist, welche das Audiosignal des Mikrofons (6) mit dem Audiosignal des Körperschallmikrofons (38) kombiniert, zur Hervorhebung eines Eigenanteils dieser beiden Audiosignale.

4. Hörsystem (2) nach einem der beiden vorhergehenden Ansprüche,
**dadurch gekennzeichnet,**
**dass** die Audiosignale der beiden Mikrofone (6) der zwei Hörgeräte (4) zu einem Kombinationssignal zusammengefasst sind, das den hervorgehobenen Eigenanteil aufweist, und dass die Steuereinheit (12) eine Audiosignalkombinationseinheit (40) aufweist, welche das Kombinationssignal mit dem Audiosignal des Körperschallmikrofons (38) kombiniert, zur weiteren Hervorhebung des Eigenanteils.

5. Hörsystem (2) nach einem der Ansprüche 2 bis 4,
**dadurch gekennzeichnet,**
**dass** das Mikrofon (6) und das Körperschallmikrofon (38) als separate Komponenten des Hörgeräts (4) ausgebildet sind.

6. Hörsystem (2) nach einem der Ansprüche 2 bis 5,
**dadurch gekennzeichnet,**
**dass** das Hörgerät (4) ein Ohrstück (34) aufweist, zum Einsetzen in einen Gehörgang des Anwenders, und das Körperschallmikrofon (38) ein Teil des Ohrstücks (34) ist.

7. Hörsystem (2) nach einem der vorhergehenden Ansprüche,
**dadurch gekennzeichnet,**
**dass** die Steuereinheit (12) zur Klassifizierung eines physiologischen Zustands (Z) ausgebildet ist, der einer Gruppe von Zuständen (Z) angehört, umfassend: Erkältung, Sturz, Schlafverhalten, soziale Aktivität, Bewegungsaktivität, Stress und Atmung.

8. Hörsystem (2) nach einem der vorhergehenden Ansprüche,
**dadurch gekennzeichnet,**
**dass** dieses einen Zusatzsensor (10) aufweist, der kein Mikrofon (6) ist und ein Zusatzsignal erfasst, welches zusätzlich zum Audiosignal zur Ermittlung des Zustands (Z) dient.

9. Hörsystem (2) nach dem vorhergehenden Anspruch,
**dadurch gekennzeichnet,**
**dass** die Steuereinheit (12) ausgebildet ist, zur Ermittlung des physiologischen Zustands (Z) ein Symptom (S1, S2, S3) des Zustands (Z) redundant zu ermitteln, nämlich auf Grundlage des Zusatzsignals sowie auf Grundlage des Audiosignals.

10. Hörsystem (2) nach einem der beiden vorhergehenden Ansprüche,
**dadurch gekennzeichnet,**
**dass** der Zusatzsensor (10) zur Messung einer Messgröße ausgebildet ist, ausgewählt aus einer Gruppe von Messgrößen, umfassend: Beschleunigung, Temperatur, elektrischer Widerstand der Haut, optische Eigenschaft der Haut und Blutsauerstoffgehalt.

11. Hörsystem (2) nach einem der vorhergehenden Ansprüche,
**dadurch gekennzeichnet,**
**dass** dieses einen weiteren Sensor, nämlich einen Zusatzsensor (10) gleicher Art aufweist, wobei vorzugsweise die beiden Sensoren jeweils ein Zusatzsensor (10) sind und jeweils eine Messungenauigkeit aufweisen und die Steuereinheit (12) ausgebildet ist, die Zusatzsignale zu verwerfen, falls eine Abweichung der Zusatzsignale der beiden Zusatzsensoren (10) größer ist als die jeweilige Messungenauigkeit..

12. Hörsystem (2) nach einem der beiden vorhergehenden Ansprüche,
**dadurch gekennzeichnet,**
**dass** die beiden Sensoren jeweils ein Zusatzsensor (10) sind und jeweils in einem der beiden Hörgeräte (4) angeordnet sind, zur Messung eines binauralen Zusatzsignals, wobei die beiden Zusatzsensoren (10) jeweils insbesondere als Beschleunigungssensor ausgebildet sind.

13. Hörsystem (2) nach einem der vorhergehenden Ansprüche,
**dadurch gekennzeichnet,**
**dass** die Steuereinheit (12) außerhalb des Hörgeräts (4) angeordnet ist und das Hörgerät (4) eine Vorverarbeitungseinheit (24) aufweist, zur Bearbeitung des Zusatzsignals und/oder des Audiosignals vor einer Weitergabe an die Steuereinheit (12).

14. Hörsystem (2) nach einem der vorhergehenden Ansprüche,
**dadurch gekennzeichnet,**
**dass** die Steuereinheit (12) eine Verstärkereinheit (16) aufweist, die mit einem Hörer (8) verbunden ist, zur Ausgabe des Audiosignals, sowie eine Analyseeinheit (18), zur Analyse des Audiosignals und zur Klassifizierung des physiologischen Zustands (Z), wobei die Verstärkereinheit (16) bevorzugt in das Hörgerät (4) integriert ist und die Analyseeinheit (18) außerhalb des Hörgeräts (4) angeordnet ist..

15. Verfahren zum Betrieb eines Hörsystems (2), insbesondere nach einem der vorhergehenden Ansprüche, für einen Anwender, wobei das Hörsystem (2) binaural ausgebildet ist, mit zwei Hörgeräten (4), insbesondere BTE-Geräten, wobei
- mittels eines jeweiligen Mikrofons (6) eines jeweiligen der Hörgeräte (4) ein jeweiliges Audiosignal erfasst wird,
- die Audiosignale an eine Steuereinheit (12) weitergegeben werden, und
- mittels der Steuereinheit (12) und auf Grundlage der Audiosignale ein physiologischer Zustand (Z) des Anwenders klassifiziert wird,
- mittels der Steuereinheit (12) auf Grundlage der Audiosignale ein Eigenanteil, der vom Anwender stammt, hervorgehoben wird und zur Klassifizierung des physiologischen Zustands (Z) verwendet wird,
wobei der physiologische Zustand (Z) bevorzugt als ein Zustand (Z) aus einer Gruppe von Zuständen (Z) klassifiziert wird, umfassend: Sturz, Schlafverhalten, soziale Aktivität, Bewegungsaktivität oder Stress..
